# EUROPEAN PATENT SPECIFICATION

(11) **EP 2 052 256 B1**
(45) Date of publication and mention of the grant of the patent: **01.02.2012**
(21) Application number: 07788437.7
(22) Date of filing: 15.08.2007
(51) Int. Cl.: G01N 33/94, G01N 33/50

(54) **THE USE OF BNP-TYPE PEPTIDES FOR THE STRATIFICATION OF THERAPY WITH ERYTHROPOIETIC STIMULATING AGENTS**
VERWENDUNG VON BNP-TYP-PEPTIDEN ZUR STRATIFIZIERUNG EINER THERAPIE MIT ERYTHROPOETISCHEN STIMULIERUNGSMITTELN
L'UTILISATION DE PEPTIDES DE TYPE BNP POUR LA STRATIFICATION DE THÉRAPIE PAR DES AGENTS DE STIMULATION ÉRYTHROPOÏÉTIQUES

(30) Priority: 17.08.2006 US 465191
(43) Date of publication of application: 29.04.2009
(73) Proprietor: F.Hoffmann-La Roche AG, 4070 Basel (CH); Roche Diagnostics GmbH, 68305 Mannheim (DE)
(72) Inventor: AMANN ZALAN, Ildiko, 69469 Weinheim (DE); MOECKS, Joachim, 68167 Mannheim (DE); BURGER, Hans Ulrich, 79395 Neuenburg (DE); ESCRIG, Cesar, 4415 Lausen (CH); SCHERHAG, Armin, 4132 Muttenz (CH); DOUGHERTY, Frank, 68480 Sondersdorf (FR); HERRMANN, Zuzana, 81545 München (DE)
(74) Representative: Huhn, Michael
(86) International application number: PCT/EP2007/058458
(87) International publication number: WO 2008/020043

(56) References cited:
- SINGH AJAY K ET AL: "Correction of anemia with epoetin alfa in chronic kidney disease" NEW ENGLAND JOURNAL OF MEDICINE, vol. 355, no. 20, November 2006 (2006-11), pages 2085-2098, XP002463404 ISSN: 0028-4793
- LEBEL MARCEL ET AL: "Hemodynamic and hormonal changes during erythropoietin therapy in hemodialysis patients" JOURNAL OF THE AMERICAN SOCIETY OF NEPHROLOGY, vol. 9, no. 1, January 1998 (1998-01), pages 97-104, XP002463405 ISSN: 1046-6673
- LIE, D.: "Cardiovascular diagnostic and treatment options" 31 July 2004 (2004-07-31), - 5 August 2004 (2004-08-05) XP002463406 Retrieved from the Internet: URL:http://www.medscape.com/viewarticle/49 9074> [retrieved on 2008-01-02]
- MAIR J ET AL: "THE IMPACT OF CARDIAC NATRIURETIC PEPTIDE DETERMINATION ON THE DIAGNOSIS AND MANAGEMENT OF HEART FAILURE" CLINICAL CHEMISTRY AND LABORATORY MEDICINE, WALTER DE GRUYTER UND CO, DE, vol. 39, no. 7, July 2001 (2001-07), pages 571-588, XP008024871 ISSN: 1434-6621

## Description

The present invention relates to diagnosing the risk of experiencing a cardiovascular complication as a consequence of medication with erythropoietic stimulating agents. The present invention also relates to optimizing the dosage of erythropoietic stimulating agents (ESAs) with respect to the risk of experiencing a cardiovascular complication.

An aim of modem medicine is to provide personalized or individualized treatment regimens. Those are treatment regimens which take into account a patient's individual needs or risks, e.g. by choosing a particular medication or a particular dosage of a medication tailored to the need of the individual patient.

Erythropoietic stimulating agents (ESAs) are administered to treat diverse disorders, particularly to treat anemia. Anemia is a common disorder, in which the number of erythrocytes (the oxygen-carrying red blood cells) is reduced. Notably, anemia is a frequent co-morbidity in cancer and kidney disease (such as e.g. in diabetes patients). Anemic patients frequently experience cardiovascular complications. These complications probably occur because the blood in anemic patients can only carry a reduced amount of oxygen, which in turn causes the heart to pump the blood more rapidly in order to satisfy the oxygen demand of the body. This may lead to left ventricular growth and left ventricular hypertrophy. Thus, anemic patients frequently experience cardiovascular complications.

It has been realized, that treatment with erythropoietin (EPO) can be used to treat anemia in patients with chronic heart failure and lead to a significant improvement in cardiac function and symptoms (van der Meer, P., Voors, A.A., Lipsic, E., et al. (2004). Erythropoietin in cardiovascular diseases. European Heart Journal, vol. 25, pp. 285-291). The benefit of EPO treatment is likely due to an increase of the number of erythrocytes, which increases the concentration of hemoglobin in the blood and thus allows the blood to carry more oxygen. Consequently, the strain on the heart is reduced, which may lead to regression of left ventricular hypertrophy and may even prevent its development.

It is known that an increase of the number of erythrocytes to abnormal levels, such as in the case of EPO abuse by athletes, can cause cardiovascular complications (Dhar, R,. Stout, C.W., Link, M.S., Homoud, M.K., et al. (2005). Cardiovascular toxicities of performance-enhancing substances in sports. Mayo Clinic Proc, vol. 80, pp. 1307-1315, erratum in Mayo Clinic Proc, January 2006, vol. 81, p. 133). In contrast to such abuse of EPO, it has previously been considered to be safe to administer ESAs at a dosage required to correct anemia up to normal or almost normal levels of hemoglobin. Such dosages or target levels of hemoglobin are currently considered to be beneficial.

However, even though medication with EPO has become an effective routine treatment of anemia, many anemic patients still die of cardiovascular complications. On the other hand, many patients with cardiovascular diseases are anemic.

BNP-type peptides (e.g. brain natriuretic peptide (BNP) and/or its N-terminal pro peptide fragment (NT-proBNP)) and their use as molecular or biochemical markers for diagnosis of certain cardiovascular complications are known as such. In WO 02/089657, it has been suggested to measure brain natriuretic peptide (BNP) to diagnose myocardial infarction. In WO 02/083913 it has been suggested to use BNP to predict near-term morbidity or mortality in patients with congestive heart failure, myocardial infarction, ST-elevated myocardial infarction, or non-ST-elevated acute coronary syndromes. However, such use does not address the problem of cardiovascular complications in patients experiencing anemia.

Therefore, there is still a need to address the problem of cardiovascular complications in patients experiencing anemia and to improve the treatment of anemia.

The object of the invention is attained by a method for diagnosing the risk of a patient of experiencing a cardiovascular complication as a consequence of medication, particularly future medication, with an erythropoiesis stimulating agent (ESA), comprising the steps of
a) measuring the level of a BNP-type peptide or a variant thereof in a sample from the patient,
b) diagnosing said risk by comparing the measured level of the BNP-type peptide or a variant thereof to at least one reference level.

The method may also comprise the step of obtaining a body fluid or a tissue sample of the patient. Preferably, the level is determined in a body fluid or tissue sample of the patient.

The invention provides methods and means, particularly markers, which allow to diagnose the risk of experiencing a cardiovascular complication as a consequence of medication with ESAs. More particularly, it has been found in the context of the invention that the measured level of a BNP-type peptide is able to indicate the degree of the risk of experiencing a cardiovascular complication as a consequence of medication with ESAs. Thus, the invention provides methods and means to identify risk patients before they receive ESA medication. Particularly, the invention allows to identify patients who have an increased cardiovascular risk if they receive a dosage of ESA sufficient to increase the hemoglobin level to normal or near-normal values. The invention can be used particularly advantageously for patients who have no known history of a cardiovascular complication. The methods and means are simple, fast, inexpensive, and suited for the use by general practitioners and/or non-cardiologists. The invention also provides corresponding uses of any of the markers, means, and methods according to the invention.

The invention also provides methods and means which allow to optimize the dosage (as defined by the target level of Hb, see definition of the dosage further below) of ESAs with respect to the cardiovascular risk. The invention provides methods and means to determine a target level of hemoglobin (and thus a dosage of ESA) that is pharmaceutically effective and at the same time avoids undesired side-effects, in particular cardiovascular complications. Thus, for each individual a more beneficial target level of hemoglobin or dosage of ESA can be determined.

In the course of the invention several findings have been made: It has been observed that ESA treatment may be associated with cause cardiovascular complications at dosages or target levels of hemoglobin which were previously considered to be therapeutically beneficial. Thus, there are cardiovascular complications which occur as a consequence of ESA medication. This observation was unexpected, as treatment with ESAs in anemic patients has previously been considered rather to reduce cardiovascular complications by reducing the strain on the heart.

Furthermore, it has been observed in the course of the invention that cardiovascular complications as a consequence of ESA medication do mainly occur in a small subset of patients. Furthermore, it has also been found that this subset of patients can be identified according to the level of a BNP-type peptide in the patient. Such patients can be considered to be risk patients. Thus, the present invention does not only draw attention to the risks associated with particular dosages of ESAs but it also provides guidance concerning which patients are at particular risk. Consequently, the present invention enables treatment with higher dosages of ESAs (or higher target levels of hemoglobin) in regular patients while it allows to identify risk patients. Such risk patients may preferably be treated with lower dosages of ESA (or lower target levels of hemoglobin) or with no ESA in order to reduce or avoid their risk of experiencing a cardiovascular complication. However, such a decision is at the discretion of the responsible physician who will als include the risk/benefit ratio for the particular patient in his consideration.

As the present invention allows the diagnosis of the risk before ESA medication commences, or before an ESA dosage is increased, the dosage of the medication can be optimized, particularly increased, to maximize the therapeutic benefit in each patient while avoiding cardiovascular complication.

Thus, the present invention allows a careful and informed decision about whether to apply ESA medication, the dosage thereof, and/or to arrange for a suitable accompanying treatment or monitoring.

The present invention is particularly advantageous to general practitioners, specialized physicians, and specialized wards, departments or clinics, which frequently have no access to extensive cardiologic examination by cardiologists. Examples are diabetes specialists, oncologists, nephrologists. Other examples are oncological, nephrological or diabetes outpatient clinics or departments. The present invention provides means and methods to such non-cardiologists for simple and reliable screening of patients for those patients who are posed at risk of experiencing a cardiovascular complication as a consequence of ESA medication.

Erythropoiesis stimulating agents are generally applied in the case disorders treatable by increasing the level of erythropoiesis, particularly in the case of anemia.

Erythropoiesis is known to the person skilled in the art. In particular, the term relates to the development of erythrocytes (the red blood cells). Due to their relatively short life-span of 120 days, the erythrocytes are continuously renewed. This renewal or development is done by proliferation and differentiation of stem cells. At present, several steps in erythropoiesis are known. Major steps are proliferation and differentiation from BFC-E cells (erythrocyte burst-forming cells, or BFC-Es (or burst-forming units, BFU-E)) via CFC-E cells (erythrocyte colony-forming cell (or colony-forming unit, CFU-E)) to the nucleus-containing erythroblasts, which in turn differentiate into erythrocytes. Interleukin-3 (IL-3) promotes the survival and proliferation of the BFC-E cells.

The erythroblasts differentiate by extruding the nucleus to become an immature erythrocyte (a reticulocyte), which then leaves the bone marrow and passes into the bloodstream. In the next day or two, the reticulocyte will lose its mitochondria and ribosomes to become a mature erythrocyte. An ESA according to the present invention may exert its influence at any stage of this process, in particular at the step of differentiation from an erythroid progenitor cell to an erythroblast. This step is controlled by signaling through the Epo receptor (EpoR). In particular, an ESA according to the invention stimulates EpoR, more particularly by binding to EpoR. An ESA according to the present invention may also stimulate the endogenous production of erythropoetin, e.g. by inhibition of hypoxia-inducible factor-prolyl hydroxylase (HIF-PH).

The term "anemia" is known to the person skilled in the art. In particular, anemia is a disorder characterized by a lack of red blood cells. Anemia can e.g. be defined by the hemoglobin content of blood. The normal hemoglobin (Hb) level for males is 14 to 18 g/dl (140 to 180 g/L); the normal level for females is 12 to 16 g/dl. If the hemoglobin level is lower than these values, the patient has anemia. Anemia according to the invention particularly relates to Hb levels of less than 14g/dl, more particularly less than 13g/dl, 11g/dl, 10g/dl, 9g/dl, 8g/dl, 7,5g/dl, 6,5g/dl, 6g/dl, 5,5g/dl, or most particularly less than 5g/dl in a malc, and to Hb levels of less than 12g/dl, more particularly less than 11g/dl, 10/dl, 9g/dl, 8g/dl, 7,5g/dl, 6,5g/dl, 6g/dl, 5,5g/dl, most particularly less than 5g/dl in a female. Moderate anemia may be considered to correspond to an Hb level of 11.0 to 12.5 g/dl. If the hemoglobin level falls below 75g/L (7,5g/dl), resting cardiac output rises significantly with an increase in both heart rate and stroke volume. The patient may complain of palpitation or a pounding pulse. Symptoms of cardiac failure may develop if the patient's myocardial reserve is reduced.

Anemia may have different causes, e.g. excessive blood loss (e.g. acute or chronic bleeding anemia), reduced or ineffective erythropoiesis, or hemolysis (hemolytic anemia, e.g. anemia due to hyperactivity of the monocyte-macrophage system, anemia due to hypersptenism or splenomegaly, immune hemolytic anemia (including autoimmune hemolytic anemia, hemoglobinuria), anemia due to mechanic destruction of erythrocytes (c.g. microangiopathic hemolytic anemia, hemolysis due to infections)).

The present invention may be applied in the context of any kind of anemia in which treatment with ESAs is able to reduce the severity of anemia. In particular, the invention can be applied in the context of anemia due to kidney disease (particularly chronic kidney disease), cancer-related anemia, and anemia due to chronic diseases (e.g. infectious or inflammatory. Cancer-related anemia may be caused by internal bleeds and/or as a side-effect of treatment with cytostatic agents. Anemia also may occur as a side-effect of cancer therapy, particularly as a side-effect of cytostatic drug therapy. Anemia is widely known as a complication in renal insufficiency, particularly diabetic nephropathy.

Erythropoiesis stimulating agents (ESAs) are known to the person skilled in the art. The term ESA comprises any kind of agent capable of stimulating erythropoiesis, particularly in vivo, more particularly in a patient. The meaning of the term stimulation is known, preferably the term relates to increasing or inducing erythropoiesis. In the context of the invention, the term ESA relates to any kind of erythropoiesis stimulating agent that is capable of increasing the level of total hemoglobin by at least 0.5 g/dl, more particularly 1 g/dl, more particularly 1.5 g/dl. Preferably, the ESA is capable of inducing such increases by a dosage usually considered reasonably safe with respect to non-cardiovascular side-effects.

The mechanism of how the ESA stimulates erythropoiesis is not particularly important in the context of the invention. For example, an ESA according to the invention may be capable of stimulating differentiation of a pluripotent stem cell in the bone marrow to an erythroblast and/or it may be capable of stimulating the differentiation from an erythroblast to an erythrocyte. However, preferably the ESA acts by stimulating EpoR or stimulating the pathway through which EpoR acts.

Examples for ESAs according to the present invention include "small molecules" as well as proteins or peptides.

"Small molecules" are understood as molecules which are not proteins, peptides, antibodies or nucleic acids, and which exhibit a molecular weight of less than 5000 Da, preferably less than 2000 Da, more preferably less than 1000 Da, most preferably less than 500 Da. Such small molecules may be identified in high throughput procedures/screening assays starting from libraries. Such methods are known in the art. Suitable small molecules can also be designed or further modified by methods known as combinatorial chemistry. An example for a small molecule is FG-2216, an inhibitor of hypoxia-inducible factor-prolyl hydroxylase (HIF-PH) (FibroGen Inc., licensed to Astellas). FG-2216 is capable of increasing the endogenous level of erythropoietin and thus to stimulate erythropoiesis. Another example may be the low-molecular weight EPO analogon PBI-1402 (ProMetic Biosciences).

Examples for ESAs according to the invention may include naturally occuring ESAs (such as erythropoetin in blood or isolated from urine, see accession no. 133170 in Online Mendelian Inheritance in Man (OMIM) on the website of the National Institutes of Health, http://www.ncbi.nlm.nih.gov/) as well as recombinantly produced ESAs, e.g. in cell culture of human or animal cells, particularly using recombinant techniques. The term "variants" is understood by the person skilled in the art and has been defined elsewhere in this specification.

Variants of ESAs are known, e.g. erythropoetin in differently glycosylated forms, e.g. due to the mode of production (e.g. the cell type of host cells used for recombinant production of a protein or peptide-based ESA).

Preferred ESAs according to the invention are erythropoietin and variants thereof Erythropoietin is a glycoprotein of 30-40 kDa molecular weight (Lee-Huang, S. (1984) Cloning and expression of human erythropoietin cDNA in Escherichia coli. Proc. Nat. Acad. Sci. vol. 81, pp. 2708-2712; Romanowski, R.R., Sytkowski, A.J. (1994). The molecular structure of human erythropoietin. Hemat. Oncol. Clin. North Am., vol. 8, pp. 885-894, 1994). Variants of erythropoietin include differently glycosylated forms of EPO and/or so-called biosimilars. Examples of variants include epoetin alpha (Amgen), epoetin beta (e.g. NeoRecormon^{™}, (Roche)), epoetin delta, epoetin-omega, darbepoetin alpha (Amgen), and Cera (Continous Erythropoiesis Receptor Activator, Hoffman-LaRoche).

The level of erythropoiesis can be measured by any method deemed appropriate by the person skilled in the art. In patients, the level of erythropoiesis can conveniently be measured e.g. by counting the number of reticulocytes and/or erythrocytes or by measuring the total level of hemoglobin. The level can also be determined by measuring the level of total packed cell volume, because the total packed cell volume is largely determined by the number of erythrocytes and can easily be measured. Such methods for measuring the level of erythropoiesis are well-known to the person skilled in the art. If the level of erythropoiesis has been determined, the degree of stimulation can be determined e.g. by comparing the level of erythropoiesis before and during medication of the ESA.

The meaning of the term "medication" is known to the person skilled in the art. In the context of the invention, the term should be understood in a broad sense comprising any kind of administration of an ESA to a patient, for example by oral administration, intravenous injection, subcutaneous injection, or intramuscular injection. Preferably, the term medication relates to long-term administration of the respective drug, e.g. an administration that causes an increase of the number of erythrocytes for at least 2 months, preferably at least 3 months, more preferably at least 6 months, more preferably at least 12 months, more preferably at least 2 years, more preferably at least 3 years. From the Examples, it can be seen that the relative difference in the risk may remain similar for an extended period of time, but that the total number of cardiovascular complications may continuously increase over time of administration. Therefore, in light of a particular clinical indication, it may be acceptable to administer an ESA for a short term (e.g. for up to 1 week, 4 weeks, 2 months or 4 months) in a risk patient identified according to the invention, whereas a decision about long-term administration in such a risk patient preferably should be based on a more careful evaluation of risk and benefit. Consequently, the invention also relates to diagnosis of the risk of experiencing a cardiovascular complication as a consequence of short-term and/or long-term medication with an ESA.

The invention takes advantage of certain biochemical or molecular markers. The terms "biochemical marker" and "molecular marker" are known to the person skilled in the art. In particular, biochemical or molecular markers are gene expression products which are differentially expressed (i.e. upregulated or downregulated) in presence or absence of a certain condition, disease, or complication. Usually, a molecular marker is defined as a nucleic acid (such as an mRNA), whereas a biochemical marker is a protein, polypeptide or peptide. The level of a suitable biochemical or molecular marker can indicate the presence or absence of the condition, disease, or complication, and thus allow diagnosis.

The present invention particularly takes advantage of BNP-type peptides as biochemical markers. Also the use of any combinations of BNP-type peptides as biochemical markers is considered in the context of the present invention.

BNP-type peptides comprise pre-proBNP, proBNP, NT-proBNP, and BNP.

The pre-pro peptide (134 amino acids in the case of pre-proBNP) comprises a short signal peptide, which is enzymatically cleaved off to release the pro peptide (108 amino acids in the case of proBNP). The pro peptide is further cleaved into an N-terminal pro peptide (NT-pro peptide, 76 amino acids in case of NT-proBNP) and the active hormone (32 amino acids in the case of BNP).

Preferred BNP-type peptides according to the present invention are proBNP, NT-proBNP, BNP, and variants thereof. BNP is the active hormone and has a shorter half-life in vivo than the inactive NT-proBNP.

Preanalytics are more robust with NT-proBNP allowing easy transportation of the sample to a central laboratory (Mueller T, Gegenhuber A, Dieplinger B, Poelz W, Haltmayer M. Long-term stability of endogenous B-type natriuretic peptide (BNP) and amino terminal proBNP (NT-proBNP) in frozen plasma samples. Clin Chem Lab Med 2004; 42: 942-4.). Blood samples can be stored at room temperature for several days or may be mailed or shipped without recovery loss. In contrast, storage of BNP for 48 hours at room temperature or at 4° Celsius leads to a concentration loss of at least 20 % (Mueller T, Gegenhuber A, et al., Clin Chem Lab Med 2004; 42: 942-4, supra; Wu AH, Packer M, Smith A, Bijou R, Fink D, Mair J, Wallentin L, Johnston N, Feldcamp CS, Haverstick DM, Ahnadi CE, Grant A, Despres N, Bluestein B, Ghani F. Analytical and clinical evaluation of the Bayer ADVIA Centaur automated B-type natriuretic peptide assay in patients with heart failure: a multisite study. Clin Chem 2004; 50: 867-73.).

Either measurement of the active or the inactive form can be advantageous, depending on the time-course of interest and the analytical equipment or storage conditions available. The most preferred BNP-type peptides according to the present invention are NT-proBNP and variants thereof.

The term "variants" in this context relates to peptides substantially similar to said peptides. The term "substantially similar" is well understood by the person skilled in the art. In particular, a variant may be an isoform or allele which shows amino acid exchanges compared to the amino acid sequence of the most prevalent peptide isoform in the human population. Preferably, such a substantially similar peptide has a sequence similarity to the most prevalent isoform of the peptide of at least 80%, preferably at least 85%, more preferably at least 90%, most preferably at least 95%. Substantially similar are also degradation products, e.g. proteolytic degradation products, which are still recognized by the diagnostic means or by ligands directed against the respective full-length peptide. The term "variants" is also meant to relate to splice variants.

The term "variant" also relates to a post-translationally modified peptide such as glycosylated peptide. A "variant" is also a peptide which has been modified after collection of the sample, for example by covalent or non-covalent attachment of a label, particularly a radioactive or fluorescent label, to the peptide.

Examples of particular variants and methods for their measurement are known (see e.g. Ala-Kopsala, M., Magga, J., Peuhkurinen, K. et al. (2004): Molecular heterogeneity has a major impact on the measurement of circulating N-terminal fragments of A-type and B-type natriuretic peptides. Clinical Chemistry, vol. 50(9), 1576-1588).

Other embodiments of the invention include the measuring of different markers in combination, simultaneously or non-simultaneously. An example is measuring of NT-proBNP in combination with BNP.

Diagnosing according to the present invention includes determining, monitoring, confirmation, subclassification and prediction of the relevant disease, disorder, complication, or risk. Determining relates to becoming aware of a disease, disorder complication, or risk. Monitoring relates to keeping track of an already diagnosed disease, disorder complication, or risk, e.g. to analyze the progression of the disease or the influence of a particular treatment on the progression of disease or disorder. Confirmation relates to the strengthening or substantiating a diagnosis already performed using other indicators or markers. Subclassification relates to further defining a diagnosis according to different subclasses of the diagnosed disease disorder, complication, or risk, e.g. defining according to mild and severe forms of the disease. Prediction relates to prognosing a disease disorder or complication before other symptoms or markers have become evident or have become significantly altered.

As will be understood by those skilled in the art, such diagnosis is usually not intended to be correct for 100% of the subjects to be diagnosed. The term, however, requires that a statistically significant portion of subjects can be diagnosed with respect to relevant disease, disorder, complication, or risk. Whether a portion is statistically significant can be determined without further ado by the person skilled in the art using various well known statistic evaluation tools, e.g., determination of confidence intervals, p-value determination, Student's t-test, Mann-Whitney test, etc.. Details are found in Dowdy and Wearden, Statistics for Research, John Wiley & Sons, New York 1983. Preferred confidence intervals are at least 90%, at least 95%, at least 97%, at least 98% or at least 99 %. The p-values are, preferably, 0.1, 0.05, 0.01, 0.005, or 0.0001.

Cardiovascular complications according to the present invention comprise any dysfunctions affecting the cardiovascular system, particularly any complications associated with plaque or thrombus formation.

Complications associated with plaque or thrombus formation the cardiovascular system are widely known. A plaque or thrombus may cause a narrowing or closure of a blood vessel and thus reduce blood flow and oxygen supply. Frequently, the clinically relevant complication is caused by a ruptured plaque or by a thrombus which is carried along with the blood stream until it gets stuck in a blood vessel, such as a coronary or pulmonary artery.

The cardiovascular complication can be a cardiac or a non-cardiac complication.

Non-cardiac complications may occur in the periphery (peripheral vascular disease), such as deep vein thrombosis, and may result e.g. in gangrene, microangiopathy, or pulmonary emboly. An example is peripheral vascular disease leading to necrosis and possibly amputation. Other examples for non-cardiac complications associated with plaque or thrombus formation include such complications affecting the brain, e.g. transient cerebral ischemic attack (TIA, a transient ischemic event in the brain) or stroke. Further examples for non-cardiac, cardiovascular complications include hypertension, hypotension.

In the cardiac system (heart and coronary arteries), complications associated with plaque or thrombus formation may include stable angina pectoris (SAP) and acute coronary syndromes (ACS). Angina pectoris is characterized by myocardial ischemia, i.e. insufficient of oxygen supply, particularly under conditions of increased oxygen demand, e.g. in the case of physical exercise. ACS patients can show unstable angina pectoris (UAP), or these individuals have already experienced a myocardial infarction (MI). Myocardial infarction is characterized by smaller or larger necrotic events (i.e. tissue destruction due to lack of oxygen supply). According to the appearance in the electrocardiogram, MI can be an ST-elevated MI or a non-ST-elevated MI. The occurring of an MI can be followed by a left ventricular dysfunction (LVD). LVD patients undergo congestive heart failure (CHF) with a mortality rate of roughly 15 %. However, the heart failure may also be acute (acute heart failure, AHF).

A cardiac complication according to the invention may also be arrhythmia, bradycardia and tachycardia.

A cardiac complication can be systolic or diastolic (i.e. primarily affecting the ejection phase (systole) or the filling phase (diastole) of the affected ventricle).

In the context of the present invention, "cardiovascular complication" particularly relates to coronary heart disease, SAP, ACS, UAP, MI (including ST-elevated MI and non-ST-elevated MI), LVD, CHF, cardiovascular death, TIA, or stroke. More particularly, "cardiovascular complication" relates to ACS, UAP, MI (including ST-elevated MI and non-ST-clevated MI), LVD, CHF, cardiovascular death, TIA, or stroke.

Cardiovascular complications can cause symptoms, which have been classified into a functional classification system according to the New York Heart Association (NYHA). Patients of Class I have no obvious symptoms of cardiovascular disease. Physical activity is not limited, and ordinary physical activity does not cause undue fatigue, palpitation, or dyspnea (shortness of breath). Patients of class II have slight limitation of physical activity. They are comfortable at rest, but ordinary physical activity results in fatigue, palpitation, or dyspnea. Patients of class III show a marked limitation of physical activity. They are comfortable at rest, but less than ordinary activity causes fatigue, palpitation, or, dyspnea. Patients of class IV are unable to carry out any physical activity without discomfort. They show symptoms of cardiac insufficiency at rest. If any physical activity is undertaken, discomfort is increased.

Accordingly, patients experiencing cardiovascular complications can be divided into individuals showing no clinical symptoms and those with symptoms (e.g. dyspnea).

Another characteristic of cardiovascular diseases can be the "left ventricular ejection fraction" (LVEF) which is also known as "ejection fraction". People with a healthy heart usually have an unimpaired LVEF, which is generally described as above 50 %. Most people with a systolic heart disease which is symptomatic generally have an LVEF of 40 % or less.

In the case of a diastolic heart disease, the LVEF may be normal (reflecting a normal diastole), whereas the filling is insufficient. Diastolic heart disease may however be characterized by increased thickness of the ventricular wall.

A cardiovascular complication according to the invention may cause symptoms, particularly symptoms according to NYHA class II-IV, more particularly according to NYHA class III-IV.

A cardiovascular complication according to the invention may be associated with an LVEF of 40% or less.

A cardiovascular complication according to the invention may either be "compensated" or "decompensated". Compensated means that the regular oxygen need of the body can still be satisfied, whereas decompensated means that the regular oxygen need of the body is not satisfied anymore.

"Experiencing a cardiovascular complication" according to the invention also includes deterioration of a pre-existing cardiovascular complication.

The term "patient" according to the present invention relates to a healthy individual, an apparently healthy individual, or, particularly, an individual suffering from a disease. Particularly, the patient is suffering from or treated for anemia, e.g. due to cancer or kidney disease (particularly chronic kidney disease, e.g. due to diabetic nephropathy). Thus, the patient may also be a patient suffering from cancer, kidney disease, or diabetes. Even more particularly, at the time of measurement or diagnosis, the patient has no known history of cardiovascular complication, and/or no or little (NYHA class I or II) symptoms of a cardiovascular complication, and/or he is not being treated for a cardiovascular complication.

Particularly, the patient is a candidate for receiving ESA medication. More particularly, at the time of measurement or diagnosis, any previous medication of the patient with an ESA, more particularly erythropoietin or a variant thereof, has taken place at least one month, particularly at least 2 months, more particularly at least 6 months, more particularly at least 1 year before. Even more particularly, at the time of measurement or diagnosis the patient has not had any previous medication with an ESA.

It is known to the person skilled in the art, under what circumstances a cardiovascular complication can be considered to occur "as a consequence" of an ESA medication. In clinical studies, a comparison of patient collectives receiving ESA medication with patient collectives not receiving ESA medication can reveal whether patients under ESA medication will experience a cardiovascular complication as a consequence of medication with ESAs: If patients receiving ESA experience cardiovascular complications more frequently, then it indicates that the increased number of cardiovascular complications occurs as a consequence of the particular ESA medication. Similarly, it can be compared whether different dosages of ESA medication cause an increased number of cardiovascular complications. Examples of such analyses are given in the Example section.

The term "as a consequence" of an ESA medication should not be interpreted as a finding that ESAs generally cause cardiovascular complications. In fact, as laid out earlier, it is usually considered that ESA medication may reduce the risk of cardiovascular complications due to reducing the strain on the heart. It is currently unknown which biological mechanism underlies the observation that ESAs (particularly at high dosages or high target levels of hemoglobin, respectively) may increase the risk of cardiovascular complications in certain patients. However, as already mentioned, it has been found in the context of the invention that the markers described in this specification are suited to detect patients at risk of experiencing cardiovascular complication if an ESA, particularly at high dosage (or high target level of hemoglobin), is administered to such patient.

Diagnosis according to the present invention is preferably done by use of a diagnostic means. A diagnostic means is any means that allows to measure the level, amount, or concentration of a substance of interest, particularly a peptide or polypeptide of interest, more particularly a BNP-type peptide.

Methods and diagnostic means which can be used to measure the levels of the respective peptides are known to the person skilled in the art. These methods include microplate ELISA-based methods, fully-automated or robotic immunoassays (available for example on Elecsys^{™} or Cobas^{™} analyzers), CBA (an enzymatic Cobalt Binding Assay, available for example on Roche-Hitachi^{™} analyzers), and latex agglutination assays (available for example on Roche-Hitachi^{™} analyzers). The methods and means for measurement also include Point-of-care devices, such as the Cardiac Reader^{™} (available from Roche Diagnostics).

Point-of-care devices are generally understood as devices which enable measuring at the patient bedside. An example is the Cardiac Reader^{™} (available from Roche Diagnostics), in combination e.g. with test strips for NT-proBNP (available as "Cardiac proBNP" from Roche Diagnostics). Such test may employ two (preferably monoclonal) antibodies directed against the peptide of interest (e.g. a BNP-type peptide). The antibodies can be identical o the antibodies used e.g. in the Elecsys^{™} or Cobas^{™} assays. E.g. the first antibody is labeled with biotin while the second antibody is labeled with gold particles. The test can be started by adding a small amount (e.g. 150 µl) of blood sample onto the test strip (e.g. into a sample well of the test strip). The erythrocytes in the sample may be separated from the remaining plasma before or after addition to the test strip, e.g. if the sample flows through a suitable fleece (e.g. a glass fiber fleece). Said separating means (e.g. fleece) is preferably part of the test strip. The antibodies (preferably already present on the test strip) are dissolved in the remaining plasma. The antibodies are capable of binding to the peptide or polypeptide of interest, forming a three-membered sandwich complex. The antibodies (bound or unbound) flow through the strip into a detection zone. The detection zone comprises means for detecting the bound complex, e.g. it may comprise streptavidin. This immobilizes the complexes and visualizes the immobilized complex as a purple line by the gold-labeled antibody. Preferably, remaining free gold-labeled antibody may then move further down the strip where it is captured in a zone comprising a synthetic peptide or polypeptide comprising the epitope of the BNP-type peptide to be detected, visualized as a separate purple line. The presence of such second line can serve as a control because it indicates that the sample flow as worked correctly and the antibody is intact, the test strip may comprise a label indicating which peptide or polypeptide of interest can be detected with the strip. It may also comprise a barcode or other code readable by a device for optical measurement of the amount of label detectable in the detection zone. Such barcode may include information indicating which peptide or polypeptide of interest can be detected with the strip. The barcode may also include lot-spcific information about the test strip.

The Cardiac Reader itself comprises a camera (e.g. charge-coupled device, CCD) that optically records the detection zone of the test strip. Signal and control lines may be identified by a pattern recognition algorithm. The intensity of the label in the signal line is typically proportional to the amount of peptide or polypeptide of interest. The optical signal may be converted into a concentration via a lot-specific calibration curve which may be stored in a code chip. The agreement of calibration code and test lot may be checked by a barcode on the test strip.

Furthermore, the person skilled in the art is familiar with different methods of measuring the level of a peptide or polypeptide. The term "level" relates to amount or concentration of a peptide or polypeptide in a patient or a sample taken from a patient.

The term "measuring" according to the present invention relates to determining the amount or concentration, preferably semi-quantitatively or quantitatively, of the nucleic acid, peptide, polypeptide, or other substance of interest. Measuring can be done directly or indirectly. Indirect measuring includes measuring of cellular responses, bound ligands, labels, or enzymatic reaction products. Preferably, measuring is carried out in vitro.

In the context of the present invention, amount also relates to concentration. It is evident, that from the total amount of a substance of interest in a sample of known size, the concentration of the substance can be calculated, and vice versa.

Measuring can be done according to any method known in the art. Preferred methods are described in the following.

In a preferred embodiment, the method for measuring the level of a peptide or polypeptide of interest, particularly a BNP-type peptide, comprises the steps of (a) contacting a cell capable of a cellular response to the peptide or polypeptide with the peptide or polypeptide for an adequate period of time, (b) measuring the cellular response.

In another preferred embodiment, the method for measuring the level of a peptide or polypeptide of interest, particularly a BNP-type peptide, comprises the steps of (a) contacting a peptide or polypeptide with a suitable substrate for an adequate period of time, (b) measuring the amount of product.

In another preferred embodiment, the method for measuring the level of a peptide or polypeptide of interest, particularly a BNP-type peptide, comprises the steps of (a) contacting a peptide or polypeptide with a specifically binding ligand, (b) (optionally) removing non-bound ligand, (c) measuring the amount of bound ligand.

Preferably, the peptide or polypeptide is contained in a sample, particularly a body fluid or tissue sample, and the amount of the peptide or polypeptide in the sample is measured.

Peptides and polypeptides (proteins) can be measured in tissue, cell, and body fluid samples, i.e. preferably in vitro. Preferably, the peptide or polypeptide of interest is measured in a body fluid sample.

A tissue sample according to the present invention refers to any kind of tissue obtained from the dead or living human or animal body. Tissue samples can be obtained by any method known to the person skilled in the art, for example by biopsy or curettage.

Body fluids according to the present invention may include blood (including derivatives thereof, e.g. blood serum or blood plasma), lymphe, cerebral liquor, saliva, and urine. Particularly, body fluids include blood, (including derivatives thereof, e.g. blood serum or blood plasma) and urine. Samples of body fluids can be obtained by any method known in the art.

For general practitioner, expensive diagnostic tools are no longer required for making cardiac diagnosis. Advantageously, using urine as sample avoids any invasive step performed on the subject. Surprisingly, it has been found in studies underlying the invention that the amount of BNP-type peptide, particularly NT-proBNP in urine is similarly valuable and reliable for diagnosis as the amount in the blood. Usually, one would expect merely minor levels of BNP-type peptide to be present in urine since it is thought that major clearance for the BNP-type peptides takes place in the kidney by glomerular filtration. Accordingly, one would not expect that (i) sufficient amounts of BNP-type peptide, particularly NT-proBNP are at all present in urine. Preferably, measurements in urine are carried out in patients without major renal complications, more preferably in patients with normal or near-normal glomerular filtration.

The term "urine" according to the present invention comprises total urine or fractions thereof. The fractions are peptide- or polypeptide-containing fractions of the urine. Samples of urine can be obtained by any method known in the art. The samples may be subjected to various known pre-treatments prior to applying the method of the present invention (i.e. processed urine samples).

Methods to obtain cell samples include directly preparing single cells or small cell groups, dissociating tissue (e.g. using trypsin), and separating cells from body fluids, e.g. by filtration or centrifugation. Cells according to the present invention comprise also platelets and other non-nuclear cells, e.g. erythrocytes.

If necessary, the samples may be further processed. Particularly, nucleic acids, peptides or polypeptides may be purified from the sample according to methods known in the art, including filtration, centrifugation, or extraction methods such as chloroform/phenol extraction.

For measuring cellular responses, the sample or processed sample is added to a cell culture and an internal or external cellular response is measured. The cellular response may include the expression of a reporter gene or the secretion of a substance, e.g. a peptide, polypeptide, or a small molecule.

Other preferred methods for measurement may include measuring the amount of a ligand binding specifically to the peptide or polypeptide of interest. Binding according to the present invention includes both covalent and non-covalent binding.

A ligand according to the present invention can be any peptide, polypeptide, nucleic acid, or other substance binding to the peptide or polypeptide of interest. It is well known that peptides or polypeptides, if obtained or purified from human or animal cells, can be modified, e.g. by glycosylation. A suitable ligand according to the present invention may bind the peptide or polypeptide also via such sites.

Preferably, the ligand should bind specifically to the peptide or polypeptide to be measured. "Specific binding" according to the present invention means that the ligand should not bind substantially to ("cross-react" with) another peptide, polypeptide or substance present in the sample investigated. Preferably, the specifically bound protein or isoform should be bound with at least 3 times higher, more preferably at least 10 times higher and even more preferably at least 50 times higher affinity than any other relevant peptide or polypeptide. In the present context, such other relevant peptides or polypeptides may be other structurally related or homologous peptides or polypeptides.

Non-specific binding may be tolerable, particularly if the investigated peptide or polypeptide can still be distinguished and measured unequivocally, e.g. according to its size on a Western Blot, or by its relatively higher abundance in the sample.

Binding of the ligand can be measured by any method known in the art. Preferably, the method is semi-quantitative or quantitative. Suitable methods are described in the following.

First, binding of a ligand may be measured directly, e.g. by NMR or surface plasmon resonance.

Second, if the ligand also serves as a substrate of an enzymatic activity of the peptide or polypeptide of interest, an enzymatic reaction product may be measured (e.g. the amount of a protease can be measured by measuring the amount of cleaved substrate, e.g. on a Western Blot).

For measurement of enzymatic reaction products, preferably the amount of substrate is saturating. The substrate may also be labeled with an detectable label prior to the reaction. Preferably, the sample is contacted with the substrate for an adequate period of time. An adequate period of time refers to the time necessary for an detectable, preferably measurably amount of product to be produced. Instead of measuring the amount of product, the time necessary for appearance of a given (e.g. detectable) amount of product can be measured.

Third, the ligand may be coupled covalently or non-covalently to a label allowing detection and measurement of the ligand.

Labeling may be done by direct or indirect methods. Direct labeling involves coupling of the label directly (covalently or non-covalently) to the ligand. Indirect labeling involves binding (covalently or non-covalently) of a secondary ligand to the first ligand. The secondary ligand should specifically bind to the first ligand. Said secondary ligand may be coupled with a suitable label and/or be the target (receptor) of tertiary ligand binding to the secondary ligand. The use of secondary, tertiary or even higher order ligands is often used to increase the signal. Suitable secondary and higher order ligands may include antibodies, secondary antibodies, and the well-known streptavidin-biotin system (Vector Laboratories, Inc.)

The ligand or substrate may also be "tagged" with one or more tags as known in the art. Such tags may then be targets for higher order ligands. Suitable tags include biotin, digoxygenin. His-Tag, Glutathion-S-Transferase, FLAG, GFP, myc-tag, influenza A virus haemagglutinin (HA), maltose binding protein, and the like. In the case of a peptide or polypeptide, the tag is preferably at the N-terminus and/or C-terminus.

Suitable labels are any labels detectable by an appropriate detection method. Typical labels include gold particles, latex beads, acridan ester, luminol, ruthenium, enzymatically active labels, radioactive labels, magnetic labels ("e.g. magnetic beads", including paramagnetic and superparamagnetic labels), and fluorescent labels.

Enzymatically active labels include e.g. horseradish peroxidase, alkaline phosphatase, beta-Galactosidase, Luciferase, and derivatives thereof Suitable substrates for detection include di-amino-benzidine (DAB), 3,3'-5,5'-tetramethylbenzidine, NBT-BCIP (4-nitro blue tetrazolium chloride and 5-bromo-4-chloro-3-indolyl-phosphate, available as ready-made stock solution from Roche Diagnostics), CDP-Star™ (Amersham Biosciences), ECF™ (Amersham Biosciences). A suitable enzyme-substrate combination may result in a colored reaction product, fluorescence or chemoluminescence, which can be measured according to methods known in the art (e.g. using a light-sensitive film or a suitable camera system). As for measuring the enyzmatic reaction, the criteria given above apply analogously.

Typical fluorescent labels include fluorescent proteins (such as GFP, YFP, RFP and derivatives thereof), Cy3, Cy5, Texas Red, Fluorescein, the Alexa dyes (e.g. Alexa 568), and quantum dots. Further fluorescent labels are available e.g. from Molcular Probes (Oregon).

Typical radioactive labels include ³⁵S, ¹²⁵I, ³²P, ³³P and the like. A radioactive label can be detected by any method known and appropriate, e.g. a light-sensitive film or a phosphor imager.

Suitable measurement methods according the present invention also include precipitation (particularly immunoprecipitation), electrochemiluminescence (electro-generated chemiluminescence), RIA (radioimmunoassay), ELISA (enzyme-linked immunosorbent assay), sandwich enzyme immune tests, electrochemiluminescence sandwich immunoassays (ECLIA), dissociation-enhanced lanthanide fluoro immuno assay (DELFIA), scintillation proximity assay (SPA), turbidimetry, nephelometry, latex-enhanced turbidimetry or nephelometry, solid phase immune tests, and mass spectrometry such as SELDI-TOF, MALDI-TOF, or capillary electrophoresis-mass spectrometry (CE-MS). Further methods known in the art (such as gel electrophoresis, 2D gel electrophoresis, SDS polyacrylamid gel electrophoresis (SDS-PAGE), Western Blotting), can be used alone or in combination with labeling or other dectection methods as described above.

Preferred ligands include antibodies, nucleic acids, peptides or polypeptides, and aptamers, e.g. nucleic acid or peptide aptamers (e.g. spiegelmers or anticalins). Methods to obtain such ligands are well-known in the art. For example, identification and production of suitable antibodies or aptamers is also offered by commercial suppliers. The person skilled in the art is familiar with methods to develop derivatives of such ligands with higher affinity or specificity. For example, random mutations can be introduced into the nucleic acids, peptides or polypeptides. These derivatives can then be tested for binding according to screening procedures known in the art, e.g. phage display.

The term "antibody" as used herein includes both polyclonal and monoclonal antibodies, as well as variants or fragments thereof, such as Fv, Fab and F(ab)₂ fragments that are capable of binding antigen or hapten. The term "antibody" also includes single-chain antibodies.

In another preferred embodiment, the ligand, preferably chosen from the group consisting of nucleic acids, peptides, polypeptides, or aptamers, is present on an array.

Said array contains at least one additional ligand, which may be directed against a peptide, polypeptide or a nucleic acid of interest. Said additional ligand may also be directed against a peptide, polypeptide or a nucleic acid of no particular interest in the context of the present invention. Preferably, ligands for at least three, preferably at least five, more preferably at least eight peptides or polypeptides of interest in the context of the present invention are contained on the array.

According to the present invention, the term "array" refers to a solid-phase or gel-like carrier upon which at least two compounds are attached or bound in one-, two- or three-dimensional arrangement. Such arrays (including "gene chips", "protein chips", antibody arrays and the like) are generally known to the person skilled in the art and typically generated on glass microscope slides, specially coated glass slides such as polycation-, nitrocellulose- or biotin-coated slides, cover slips, and membranes such as, for example, membranes based on nitrocellulose or nylon.

The array may include a bound ligand or at least two cells expressing each at least one ligand.

In another preferred embodiment, the ligand, preferably chosen from the group consisting of nucleic acids, peptides, polypeptides, antibodies and aptamers, is present on a solid support, preferably an array. According to the present invention, the term "array" (including "gene chips", "protein chips", antibody arrays and the like) refers to a solid-phase or gel-like carrier upon which at least two compounds are attached or bound in one-, two- or three-dimensional arrangement. Solid supports or arrays comprising a ligand or binding, agent for a BNP-type peptide are well known in the art and include, inter alia, commercially available column materials, polystyrene beads, latex beads, magnetic beads, colloid metal particles, glass and/or silicon chips and surfaces, nitrocellulose strips, membranes, sheets, duracytes, wells and walls of reaction trays, plastic tubes etc. The ligand or agent may be bound to many different carriers. Examples of well-known carriers include glass, polystyrene, polyvinyl chloride, polypropylene, polyethylene, polycarbonate, dextran, nylon, amyloses, natural and modified celluloses, polyacrylamides, agaroses, and magnetite. The nature of the carrier can be either soluble or insoluble for the purposes of the invention. Suitable methods for fixing/immobilizing said ligand or agent are well known and include, but are not limited to ionic, hydrophobic, covalent interactions and the like.

It is also contemplated to use "suspension arrays" as arrays according to the present invention (Nolan JP, Sklar LA. (2002). Suspension array technology: evolution of the flat-array paradigm. Trends Biotechnol. 20(1):9-12). In such suspension arrays, the carrier, e.g. a microbead or microsphere, is present in suspension. The array consists of different microbeads or microspheres, possibly labeled, carrying different ligands.

The invention further relates to a method of producing arrays as defined above, wherein at least one ligand is bound to the carrier material in addition to other ligands.

Methods of producing such arrays, for example based on solid-phase chemistry and photo-labile protective groups, are generally known (US 5,744,305). Such arrays can also be brought into contact with substances or substance libraries and tested for interaction, for example for binding or change of confirmation. Therefore, arrays comprising a peptide or polypeptide as defined above may be used for identifying ligands binding specifically to said peptides or polypeptides.

Thus, the invention also relates to the use of a diagnostic means capable of measuring, preferably in vitro, a patient's level of a BNP-type peptide, particularly NT-proBNP, for diagnosing the patient's risk of experiencing a cardiovascular complication as a consequence of future medication with an ESA.

The invention also relates to the use of a kit comprising a means capable of measuring, preferably in vitro, a patient's level of a BNP-type peptide, particularly NT-proBNP, for diagnosing the patient's risk of experiencing a cardiovascular complication as a consequence of future medication with an ESA.

Also disclosed is a kit comprising a means or an agent for measuring a BNP-type peptide. Such means or agent may be any suitable means or agent known to the person skilled in the art. Examples for such means or agents as well as methods for their use have been given in this specification. For example, a suitable agent may be any kind of ligand or antibody capable of specifically binding to a BNP-type peptide. The kit may also comprise any other components deemed appropriate in the context of measuring the level(s) of the respective biomarkers, such as suitable buffers, filters, etc.

Optionally, the kit may additionally comprise a user's manual for interpreting the results of any measurement(s) with respect to diagnosing the risk of a patient of experiencing a cardiovascular complication as a consequence of ESA medication, particularly future ESA medication. Particularly, such manual may include information about which measured level corresponds to which grade of risk. This is outlined in detail elsewhere in this specification. Additionally, such user's manual may provide instructions about correctly using the components of the kit for measuring the level(s) of the respective biomarker.

The invention also relates to the use of said kit for diagnosing the risk of a patient of experiencing a cardiovascular complication as a consequence of medication, particularly future medication, with an ESA. The present invention also relates to the use of said kit in any of the methods according to the present invention for diagnosing the risk of a patient of experiencing a cardiovascular complication as a consequence of ESA medication, particularly future ESA medication.

Moreover, disclosed is a device for diagnosing the risk of a patient of experiencing a cardiovascular complication as a consequence of future medication with an ESA, comprising
a) means for measuring the amount of a BNP-type peptide in a sample from the patient; and
b) means for diagnosing said risk by comparing the measured level to at least one reference level.

### The above-described device as such is not apart of the present invention.

The present invention also relates to the use of such device for diagnosing the risk of a patient of experiencing a cardiovascular complication as a consequence of future medication with an ESA.

The term "device" as used herein relates to a system of means comprising at least the aforementioned means operatively linked to each other as to allow diagnosis of heart failure. Preferred means for measuring the level of a BNP-type peptide and for diagnosing the risk are disclosed elsewhere in this specification in connection with the method of the invention. How to link the means in an operating manner will depend on the type of means included into the device. For example, where means for automatically measuring the level of the BNP-type peptide are applied, the data obtained by said automatically operating means can be processed by, e.g., a computer program in order to diagnose the risk. Preferably, the means are comprised by a single device in such a case. Said device may accordingly include an analyzing unit for measuring the level of the BNP-type peptide in an applied sample and a computer unit for processing the resulting data for the diagnosis. Alternatively, where means such as test stripes are used for measuring the level of the BNP-type peptide, the means for diagnosing may comprise control stripes or tables allocating the measured level to a level known to be accompanied with heart failure or a level known to be indicative for a healthy subject as discussed above. The test stripes are, preferably, coupled to a ligand or agent which specifically binds to the BNP-type peptide. The strip or device, preferably, comprises means for detection of the BNP-type peptide binding to the said ligand or agent. Preferred means for detection are disclosed in connection with embodiments relating to the method of the invention above. In such a case, the means are operatively linked in that the user of the system brings together the result of the determination of the amount and the diagnostic value thereof due to the instructions and interpretations given in a manual. The means may appear as separate devices in such an embodiment and are, preferably, packaged together as a kit. The person skilled in the art will realize how to link the means without further ado. Preferred devices are those which can be applied without the particular knowledge of a specialized clinician, e.g., test stripes or electronic devices which merely require loading with a sample. The results may be given as output of a diagnostic parameter or raw data which need interpretation by the clinician. Further preferred devices comprise the analyzing units/devices (e.g., biosensors, arrays, solid supports coupled to ligands or agents specifically recognizing BNP, Plasmon surface resonace devices, NMR spectrometers, mass- spectrometers etc.) or evaluation units/devices referred to above in accordance with the method of the invention.

The method according to the present invention comprises the step of diagnosing the risk of the patient by comparing the measured level of the BNP-type peptide to at least one reference level, e.g. to known levels associated with different grades of risk in a patient.

According to the present invention, the term "risk" relates to the probability of a particular incident, more particularly a cardiovascular complication, to take place. For example, a risk can be that the given incident is going to take place with a probability of at least 2%, 5%, 10%, 15% or 20% in a given patient within a particular time, e.g. 500, 750, 1000, or 1250 days. Clinical studies can provide data indicating such risks. The given risk can be derived e.g. from a suitable Kaplan-Meier plot for time to a given incident see e.g.. Example 3 and also the particular risks and hazard rations mentioned therein.
Although the risk can be expressed in absolute values, it may frequently be more useful to express the risk in relative terms, e.g. in terms of a increased or highly increased risk relative to a particular given risk. The person skilled in the art is very familiar with such relative terms. For example, there is an average given risk of experiencing a cardiovascular complication in the general population. However, it may be more relevant to know, whether a particular patient has an additional risk of experiencing a cardiovascular complication, so that the total risk of this patient is "increased". Advantageously, the present invention also allows to diagnose such a relative risk.

A relative risk can be expressed in terms of hazard ratios. The term "hazard ratio" is known to the person skilled in the art. It can express the relation of the risk between two subgroups e.g. the hazard ratio between a high dosage group and the low dosage group. Another example is a hazard ratio between a high dosage group versus a low dosage group for a low level of BNP-type peptide versus for a high level of BNP-type peptide. A difference in the hazard ratios is known as interaction to be extracted from interaction models, e.g. of dosage groups with certain BNP-levels. The terms interaction and interaction model are known to the person skilled in the art.

Particularly, the present invention allows to identify patients at increased risk of experiencing a cardiovascular complication if treated with a high dosage of an ESA (i.e. aimed at a high target level of hemoglobin), respectively.

For example, the risk can be increased, or highly increased. The risk can also not be increased. The person skilled in the art is familiar with the meaning of these terms. For example, if a particular patient has a higher risk than an average patient, then the person skilled in the art will usually designate such risk as "increased". Preferably, the term "increased risk" is understood as indicating that the patient should not be treated with a high dosage of an ESA. Preferably, a patient having an increased risk should be monitored with additional care concerning the development of any cardiovascular complications. The person skilled in the art will understand that the final decision about treatment will be with the responsible physician who will consider additional relevant factors, such as the age of the patient, family history of cardiovascular complication, the availability of monitoring possibilities etc.

In the context of the invention, an increased risk of experiencing a cardiovascular complication as a consequence of administration of an ESA particularly relates to an increase of the risk of at least by 1.5 times, 2 times, 3 times, 3,5 times or 4 times as compared to the risk of an average patient, preferably of an average patient of the same age and gender, more preferably to a patient of same age, gender and disorder causing symptoms treatable with ESA medication (e.g. diabetes or cancer. Further disorders causing symptoms treatable with ESA medication have already been mentioned elsewhere in this specification).

The person skilled in the art is able to determine known levels of BNP-type peptides which are associated with different grades of risk of experiencing a cardiovascular complication as a consequence of ESA medication.

According to the invention, the higher the measured level of the BNP-type peptide, the higher is the risk of experiencing a cardiovascular complication.

Preferably, the risk is determined by comparing the measured level of the BNP-type peptide to a reference level. The term "reference level" is known to the person skilled in the art. Particularly, a reference level may be associated with a particular risk or it may distinguish between different grades of risk. It will be appreciated that the reference level may also be chosen according to the desired sensitivity or specificity of diagnosis. A higher sensitivity means that a higher fraction of all patients having a particular diagnosis are identified and/or that less patients having a particular diagnosis are misdiagnosed as not having the diagnosed disease, complication, or risk. A higher specificity means that a higher fraction of the patients identified as having a particular diagnosis do indeed have the diagnosed disease, complication, or risk. The higher the desired sensitivity for a particular diagnosis, the lower is the specificity of this diagnosis and vice versa. Therefore, the reference level may be chosen by the person skilled in the art according to the desired sensitivity and specificity.

In the context of this discussion, it is evident that a reference level may not only be a single value, but it may also include a range of values.

More particularly, reference levels can be derived from levels of BNP-type peptides determined e.g. in clinical studies such as presented in the examples.

Examples for reference levels are given below, namely plasma levels of NT-proBNP are given which have been found in the course of the invention to be associated with or distinguishing the indicated grades of risk of experiencing a cardiovascular complication as a consequence of ESA medication.

It is evident, that the levels given below can serve only as a first classification of the risk of a patient. For example, the risk may also be dependent on the spare pumping capacity of heart of the particular patient.

According to the invention, e.g. a level corresponding to a plasma level of less than 500 pg/ml, more particularly less than 400 pg/ml, most particularly less than 300 pg/ml of NT-proBNP is associated with no increased risk of experiencing a cardiovascular complication as a consequence of ESA medication.

According to the invention, e.g. a level corresponding to a plasma level of equal or more than 300 pg/ml, more particularly equal or more than 400 pg/ml, most particularly equal or more than 500 pg/ml of NT-proBNP is associated with an increased risk of experiencing a cardiovascular complication as a consequence of ESA medication.

According to the invention, e. g. a level corresponding to a plasma level of equal or more than 1000pg/ml of NT-proBNP may indicate a highly increased risk at which it may be reconsidered whether the patient should be treated with ESA at all.

It is evident that the given levels may overlap, depending on the chosen sensitivity and specificity. Therefore, according to the invention, e.g. a level corresponding to a plasma level of 300 to 500 pg/ml, more particularly 350 to 450 pg/ml, more particularly 380 to 420 pg/ml, particularly of 400 pg/ml of NT-proBNP is able to distinguish between no increased risk and increased risk of experiencing a cardiovascular complication as a consequence of ESA medication. If the measured level is higher than this distinguishing level, then the measured level indicates an increased risk of experiencing a cardiovascular complication as a consequence of ESA medication. Such distinguishing level may also be called "cut-off" or "decision threshold".

Once the risk in a patient has been diagnosed, it may have consequences for the subsequent treatment as described below. The grades of risk mentioned below particularly refer to the grades of risk associated with the above described levels ofNT-proBNP.

If a method according to the present invention indicates no increased risk, then treatment may be continued as planned, e.g. a high dosage of ESA may be administered (or correspondingly it may be aimed at a high target level of ESA). The ESA treatment my be accompanied by monitoring the NT-proBNP a levels at loosely spaced intervals, e. g. every 4 weeks, 2 months, or 3 months.

The reference level may be adapted for particular groups of patients. For example, the reference level for measurement in blood samples may be increased by 10-30 %, more preferably 15-25%, more preferably 20 % for patients suffering from renal disease.

If a method according to the present invention indicates an increased risk, then treatment may be adapted. Preferably, treatment will be accompanied by further measuring of the level of the BNP-type peptides of the invention and by further diagnosis, such as clectrocardiography, echocardiography, or any other suitable methods known to the skilled cardiologist. The dosage of ESA medication may be reduced or no ESA medication may be administered. Therefore, the present invention also provides a method of treating a patient who is receiving or about to receive ESA medication.

As already mentioned, the present invention also allows to determine an optimized dosage of medication with an ESA, particularly with respect to the cardiovascular risk of the patient. Therefore, the present invention also relates a method for determining the dosage of medication, preferably future medication, of an ESA to be administered to a patient particularly with respect to the risk of the patient of experiencing a cardiovascular complication as a consequence of said ESA medication, comprising the steps of (a) measuring, preferably in vitro, the level of a BNP-type peptide in the patient, (b) determining the dosage to be administered to the patient by comparing the measured level of the BNP-type peptide to at least one reference level distinguishing between different dosages to be administered.

If a dosage of medication with an ESA is chosen, it is generally desirable to choose the highest possible dosage that does achieve efficient treatment of anemia but dose not cause undesired side-effects. However, if the responsible physician is not aware of the risk of a particular undesired side-effect, such as cardiovascular complication, then he may accidentally choose a dosage which is too high and may result in a cardiovascular complication. In contrast, if the physician can not determine a given risk, then he may choose a dosage which is too low to reach optimal therapeutic benefit, particularly efficient treatment of anemia.

Preferably, the dosage should be high enough to sufficiently treat anemia, in order to reduce the strain on the heart. Preferably, the dosage should be low enough to avoid cardiovascular complication resulting from the ESA medication.

Advantageously, the present invention provides (1) information that there is a risk of experiencing a cardiovascular complication as a consequence of ESA treatment, and (2) information regarding optimizing the dosage of the ESA (particularly according to the target hemoglobin level) in order to minimize the risk of cardiovascular complication.

The invention (3) also provides information of whether ESA treatment may be started in cases of relatively mild anemia, i.e. already at relatively high levels of Hb in a patient (e.g. at 11.0 to 12.5 g/dl) ("early treatment") or rather only in slightly more severe anemia (e.g. less than 10.5 g/dl, or at 9.5 to 10.5 g/dl Hb) ("late treatment").

More particularly, if the level of the BNP-type peptide indicates an increased risk of experiencing a cardiovascular complication (such as described elsewhere in this specification), then it indicates that the patient should not be treated with a high dosage of an ESA and/or it indicates that the patient may only be treated with a low dosage of an ESA and/or it indicates that treatment should not be initiated early. It may also indicate that the patient should not be treated with an ESA. It may also indicate that the patient should additionally be treated as described for the case of an increased risk, preferably the patient should additionally be further monitored as described elsewhere.

In the context of the invention, the terms "low dosage" and "high dosage" of an ESA are preferably understood as dosages required to increase the hemoglobin level to a certain target value. Thus, the terms "low dosage" and "high dosage" may also be understood as relating to a "partial" versus a "full" correction of anemia. A low dosage preferably relates to an ESA dosage not higher than required to reach a Hb level of 13 g/dl, more preferably a dosage not not higher than required to reach a Hb level of 12 g/dl. Examples for low dosages may include ESA dosages aimed at reaching a target Hb level of 9, 9.5, 10, 10.5, 11, 11.5, 12, 12.5, or 13 g/dl. A high dosage preferably relates to an ESA dosage sufficient to reach a Hb level of at least 14 g/dl, more preferably a dosage sufficient to reach a Hb level of 14.5 g/dl. Examples for high dosages may include ESA dosages aimed at reaching a target Hb level of 14, 14.5, 15 g/dl.

Alternatively, the term "low dosage" and "high dosage" may be understood as dosages capable of increasing the Hb level by less than 2 g/dl particularly by less than 1,5 g/dl, 1 g/dl, or 0,5 g/dl (low dosages) or by at least 2 g/dl, 2,5 g/dl, or 3 g/dl.(high dosages).

For example, a high dosage may correspond to a dosage sufficient to maintain a target level of 13 to 15 g/dl hemoglobin in a patient with a level of 11.0 to 12.5 g/dl hemoglobin before onset of ESA medication. In another example, a low dosage may correspond to a dosage sufficient to maintain a target of 10.5 to 11.5 g/dl hemoglobin in a patient with a level of less than 10.5 g/dl hemoglobin, preferably with a level between 9.5 and 10.5g/dl, before onset of ESA medication.

The invention provides means and methods regarding diagnosis of the risk of experiencing a cardiovascular complication as a consequence of ESA treatment. In particular, the teaching of the invention relates to ESA dosages previously considered not to be associated with the risk of experiencing a cardiovascular complication, e.g. a dosage which is not higher than required to reach a hemoglobin concentration in blood of at least 17, 18, 19, or 20 g/dl. Thus the teaching of the present invention relates already to dosages which do not cause hyperviscosity of the blood or any other adverse events known in the context of blood doping in athletes.

It will be appreciated that the above given values for "low" or "high" dosage do not relate to dosages required in healthy individuals (in which no ESA may be required at all to reach said levels), but to dosages administered to patients suffering from anemia. In particular, the levels relate to patients having an Hb level (before ESA treatment commences) of less than 12g/dl, particularly less than 10 g/dl, more particularly less than 9 g/dl, most particularly less than 8 g/dl in a male patient, or to an Hb level (before ESA treatment commences) of less than 11g/dl, particularly less than 10 g/dl, more particularly less than 9 g/dl, most particularly less than 8 g/dl in a female patient.

The person skilled in the art is familiar with methods to determine ESA dosages in order to reach a certain level or target value of Hb. For example, in an anemic patient (e.g. having an Hb level of 9.5 to 12.5 g/dl) treatment may be initiated with a dosage corresponding to 2000 international units of NeoRecormon^{™}, administered subcutaneously once a week. The Hb level in such patient would be measured e.g. after a week or after a month and the dosage would be further adapted until a treatment regimen is found that meets a given target value. There may be further adapting and monitoring of the dosage, e.g. depending on disease progression. It will be appreciated that the actually reached Hb levels may fluctuate to a certain degree, e.g. depending on the frequency of administration of the ESA or the progression of the disease.

Any preferred embodiments or features mentioned in this description do of course apply correspondingly to the aspect of determining of the dosage of ESA medication.

The invention also relates to monitoring the risk of experiencing a cardiovascular complication as a consequence of medication, particularly future medication, with an ESA. Furthermore, the invention also relates to further monitoring the risk once the risk it has been diagnosed. More particularly, the monitoring relates to monitoring in a patient in whom at the time of such first measurement or diagnosis any previous medication with an ESA, more particularly erythropoietin or a variant thereof, has taken place at least one month, particularly at least 2 months, more particularly at least 6 months, more particularly at least 1 year before the time of measurement or diagnosis. Even more particularly, at the time of first measurement or diagnosis the patient has not had any previous medication with an ESA. The invention also relates to monitoring the current cardiovascular status in any such patient.

The term "monitoring" is known to the person skilled in the art and has been defined elsewhere in this specification. More particularly, monitoring may be carried out by diagnosing the risk of the patient in regular intervals, for examples at intervals of approximately 2 hours, 10 hours, 1 day, 2 days, 3 days, 4 days, 1 week, 2 weeks, 3 weeks, 4 weeks, 1 month, , 2 months, 4 months, 6 months, or 1 year. The term "approximately" in this context is understood by the person skilled in the art. Therefore, the actual interval may also deviate from an intended regular interval depending on practical circumstances such as arranging for suitable appointments etc.. Particularly the interval may e.g. deviate by up to 100%, preferably up to 50%, more preferably up to 25%,more preferably up to 10%. The monitoring may be carried out accompanying medication with an ESA nor not accompanying treatment with an ESA. The monitoring also allows to further adapt and optimize the dosage of an ESA medication.

Any preferred embodiments or features mentioned in this description do of course apply correspondingly to the aspect of monitoring.

In another embodiment, the present invention relates to a method for deciding on treatment of a patient with ESA medication and/or for deciding about the dosage of a planned ESA medication, comprising the steps of (a) measuring, preferably in vitro, the level of a BNP-type peptide, (b) diagnosing the risk of the patient of experiencing a cardiovascular complication as a consequence of ESA medication by comparing the measured level of the BNP-type peptide to known levels associated with different grades of risk in a patient, (c) optionally initiating an examination of the patient by a cardiologist, (d) recommending the initiation of the treatment or refraining from the treatment, optionally in consideration of the result of the patient's examination by the cardiologist. Preferably, initiating an examination by a cardiologist and/or refraining from treatment is recommended if the method indicates the presence of a an increased risk of experiencing a cardiovascular complication as a consequence of medication with an ESA. It is evident that the method may be adapted according to all embodiments or preferred aspects of the invention mentioned in this specification.

The following examples illustrate the invention and are not intended to limit its scope in any way.

Finally, the present invention also encompasses the use of the devices described herein above or BNP-type peptide or variant thereof for diagnosing the risk of a patient of experiencing a cardiovascular complication as a consequence of future medication with an ESA.

### Description of the Figures:

Figure 1: Kaplan-Meier graphs and risk ratios (HR, hazard ratios) for the primary cardiovascular endpoint.
Figure 2: Kaplan-Meier graphs and risk ratios (HR, hazard ratios) for cardiac adverse events (cardiac comlications).
Figure 3: Kaplan-Meier graphs and risk ratios (HR, hazard ratios) for cardiovascular adverse events (AE) (cardiovascular complications).
Figure 4: Graphical display of estimated risk ratios (HR, hazard ratios) for cardiovascular adverse events (CVAE) and the primary cardiovascular endpoint (P Endp) including 95% confidence intervals.

### Example 1

### Measurement of NT-proBNP:

NT-proBNP is determined by an electrochemoluminescence immunoassay (Elecsys proBNP sandwich immunoassay; Roche Diagnostics, Mannheim, Germany) on Elecsys 2010. The assay works According to the electrochemoluminescence sandwich immunoassay principle. In a first step, the biotin-labelled IgG (1-21) capture antibody, the ruthenium-labclled F(ab')₂ (39-50) signal antibody and 20 microliters of sample are incubated at 37 °C for 9 minutes. Afterwards, streptavidin-coated magnetic microparticles are added and the mixture is incubated for additional 9 minutes. After the second incubation, the reaction mixture is transferred to the measuring cell of the system where the beads are magnetically captured onto the surface of an electrode. Unbound label is removed by washing the measuring cell with buffer.

In the last step, voltage is applied to the electrode in the presence of a tri-propylamine containing buffer and the resulting electrochemoluminescent signal is recorded by a photomultiplier. All reagents and samples are handled fully automatically by the Elecsys^{®} instrument. Results are determined via a calibration curve which is instrument-specifically generated by 2-point calibration and a master curve provided via the reagent barcode. The test is performed according to the instructions of the manufacturer.

### Example 2

### Obtaining of samples:

Blood for BNP-type peptide analysis is sampled in EDTA-tubes containing 5000 U aprotinine (Trasylol, Beyer, Germany) and Lithium-Heparin-tubes (for clinical chemistry), as appropriate. Blood and urine samples are immediately spun for 10 min. at 3400 rpm at 4 °C. Supernatants are stored at -80 °C until analysis.

### Example 3

### Objective:

The CREATE study was an open, randomized, parallel group, multi-center study to investigate the effect of early anemia correction with epoetin beta on the reduction of cardiovascular risk in patients with chronic renal anemia who are not on renal replacement therapy. The primary objective of the study was to investigate the effect of early epoetin beta treatment to a target hemoglobin (Hb) level of 13-15 g/dL on cardiovascular morbidity and compare these effects with those attained with epoetin beta treatment to maintain a target Hb level of 10.5-11.5 g/dL.

### Method:

The primary endpoint was the combined endpoint of all protocol-specified cardiovascular events (time to first event): angina pectoris leading to hospitalization for at least 24 hrs or prolongation of hospitalization, acute heart failure, fatal or non-fatal myocardial infarction, fatal or non-fatal stroke, sudden death, transient cerebral ischemic attack (TIA), peripheral vascular disease (amputation, necrosis), cardiac arrhythmias leading to hospitalization for at least 24 hrs or prolongation of hospitalization.

### Secondary variables:

- cardiovascular mortality, all cause mortality, chronic heart failure (NYHA classification), cardiovascular interventions: time to event and frequency of episodes
- hospital admissions and duration of hospitalizations for cardiovascular reasons
- left ventricular mass index, left ventricular volume, left ventricular ejection fraction, fractional myocardial shortening
- Quality of Life (SF-36 questionnaire)

In the NT-proBNP sub study additional lab measurements were taken at baseline, at 6,12, 24, 36 and 48 month for a subset of patients in the CREATE study. Measurements taken are NT-proBNP and some other lab measurements.

The entire study cohort was followed for the occurrence of adverse events including cardiac and cardiovascular (CV) adverse events. All CV events were classified by professional Roche drug safety staff into MedDRA (Medical Directionary of Regulatory Activities) categories. Cardiac Events comprised specific events like angina pectoris, arrhythmias, acute or chronic heart failure, myocardial infarction, bradycardia and tachycardia. Cardiovascular events contain in addition events related to the vascular system such as hypertension, hypotension, peripheral vascular disease or deep vein thrombosis. It is typical for adverse events recordings that also non-specific disorders were noted under cardiovascular events such as palpitations or flush.

### Analysis:

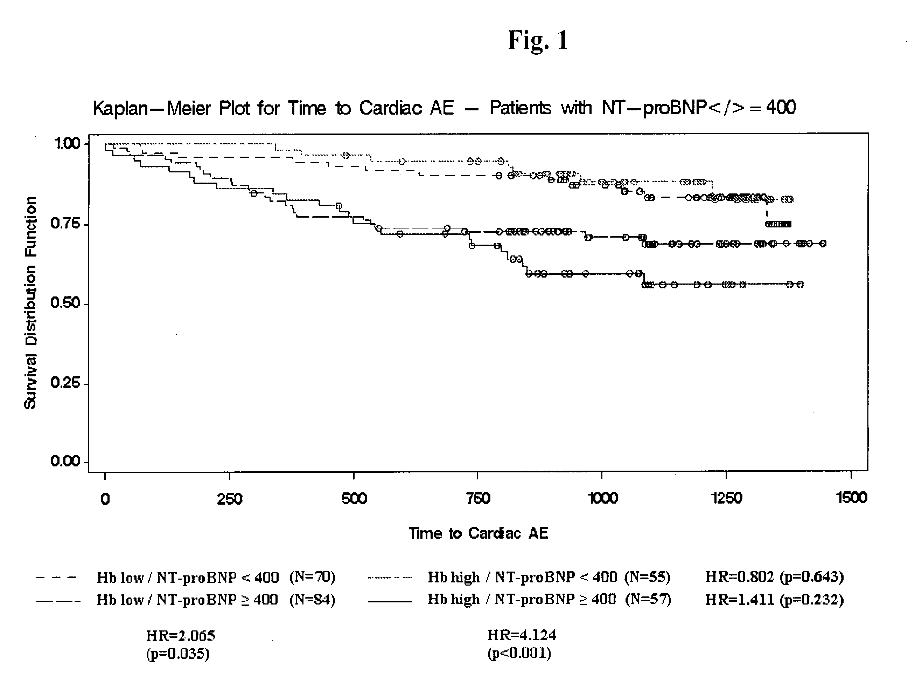

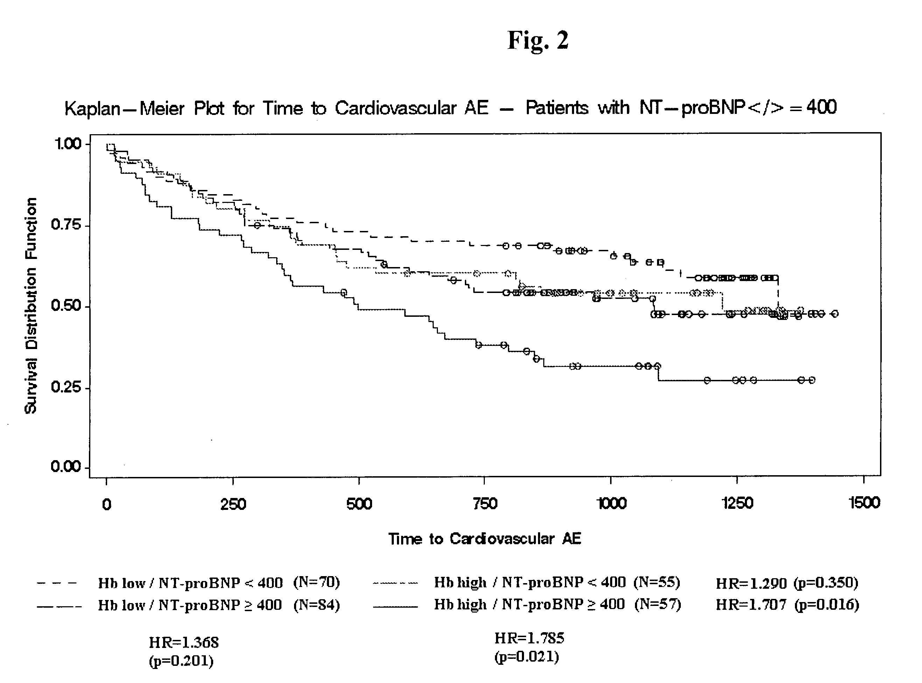

In a first preliminary analysis baseline NT-proBNP levels of 266 patients of the CREATE study population were stratified into the 2 treatment groups ("Hb high" = Hb target value 13-15g/dL and "Hb low" =Hb target value 10.5-11.5 pg/dL). Furthermore the groups were divided by the preliminary median NT-proBNP level of the entire cohort (> and < 400 pg/ml).The time to first cardiac event as defined by the primary endpoint of the study was plotted in a Kaplan Meier graph (e.g. fraction of the population who have not experienced this particular event) Fig. 1. Hazard ratios (i.e. factor by which the respective risk is increased or decreased) were calculated and p values < 0.05 were considered significant. Equally Kaplan-Meier plots for time to first cardiac adverse event and cardiovascular event were generated (Fig.2-3).

### Preliminary Results:

An overview of the incidence rates (ignoring the timing of the events) of the 2 treatment groups is provided by the following table:

| | **CV AE** | | **primary CV-endpoint** | |
|---|---|---|---|---|
| | **NT-** | **NT-** | **NT-** | **NT-** |
| | **proBNP<400** | **proBNP≥400** | **proBNP<400** | **proBNP≥400** |
| **Hb low** | 47% | 49 % | 11% | 21 % |
| **Hb high** | 40% | 68% | 11% | 35% |

In the patients group with NT-proBNP baseline levels below 400pg/ml no difference in incidence with respect to the *primary CV endpoint* or *cardiovascular adverse events* is apparent for the 2 treatment groups, whereas those with baseline values above 400 pg/ml show higher incidences in particular for the 'Hb high' group. A closer analysis by 'time-to-event' methods showed that a statistically significant higher risk of experiencing the *primary candiovascular endpoint* was observed in the "Hb high" treatment group (>3,7 fold) and a strong trend for the "Hb low" group, just not reaching statistical significance (Fig1).

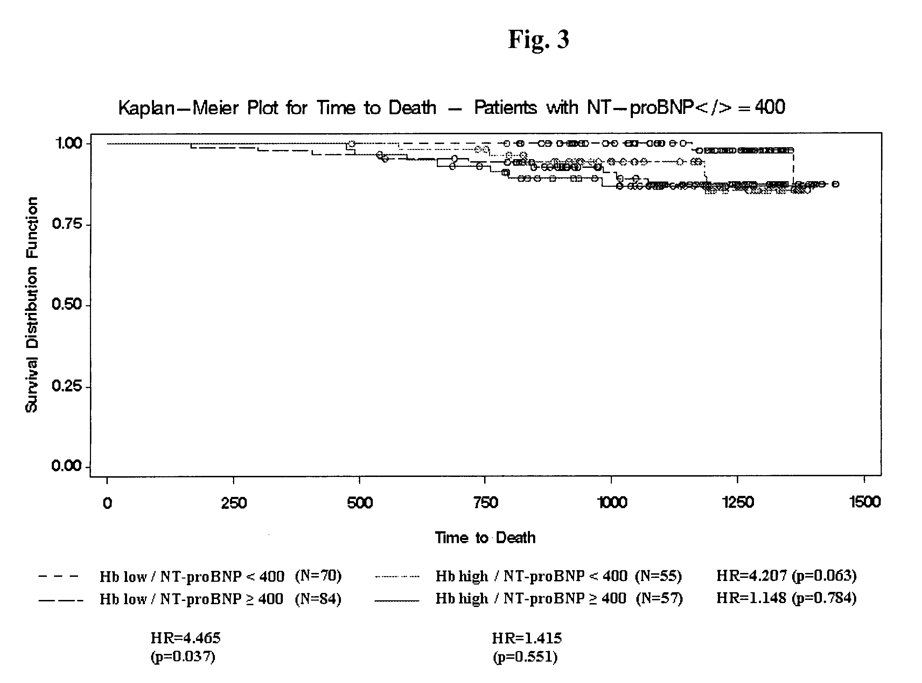

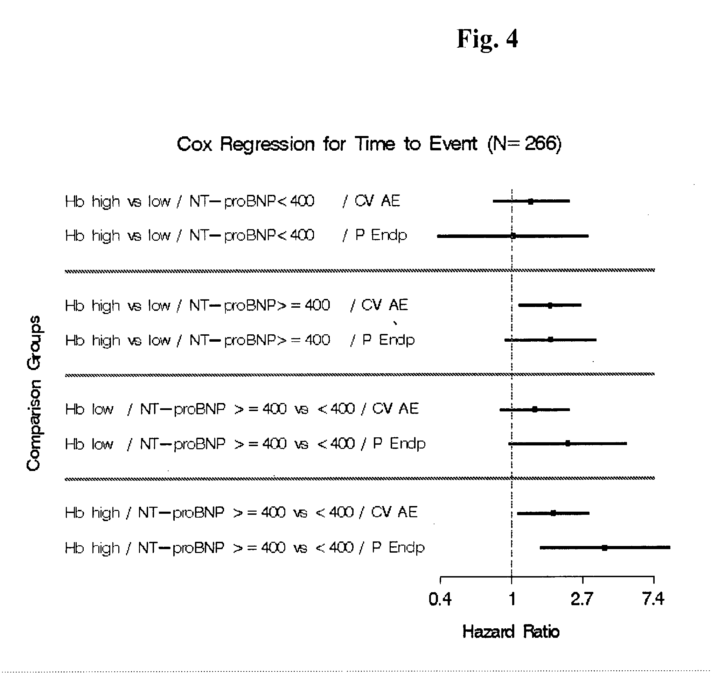

Patients with values above 400 pg/ml compared to those with values below 400 pg/ml experienced statistically significantly more *cardiac events* in both treatment strategy groups with a higher risk ratio for the "Hb high" group (Fig 2). For *cardiovascular adverse events* the risk was statistically significant higher in the group that was treated with the high target value strategy than in the low target value strategy (patient group with baseline values >400 pg/ml) (Fig 3). The results of the risk analysis for the *primary endpoint* and *cardiovascular adverse events* are summarized in the forest plot (Fig 4), which shows the estimated risk ratios together with confidence intervals.

### Conclusion:

Preliminary results indicate that NT-proBNP could identify a patient population (baseline levels below 400 pg/ml) within the CREATE population that can be treated safely with epoetin beta with the low and high target Hb strategy. In patients with NT-proBNP levels above 400 pg/ml at baseline the marker identifies a group of patients at a higher risk for cardiac and cardiovascular events. The cut-off 400 pg/ml is a preliminary choice and there is the possibility of other choices depending on clinical decision situation. A careful risk benefit analysis in particular for the high target Hb strategy is indicated, including a close monitoring of the CV-status for this treatment strategy.

## Claims

1. A method for diagnosing the risk of a patient of experiencing a cardiovascular complication as a consequence of future medication with an erythropoiesis stimulating agent (ESA), comprising the steps of
a) measuring the level of a BNP-type peptide in a sample from the patient,
b) diagnosing said risk by comparing the measured level of the BNP-type peptide or a variant thereof to at least one reference level.

2. The method according to claim 1, wherein step a) and/or step b) are carried out before medication with the ESA commences and/or wherein the patient has not yet received medication with an ESA.

3. The method according to any of claims 1 to 2, wherein the BNP-type peptide is BNP, NT-proBNP.

4. The method according to claim 3, wherein the BNP-type peptide is NT-proBNP.

5. The method according to any of claims 1 to 4, wherein the reference level corresponds to a plasma level of NT-proBNP of 300 to 500 pg/ml.

6. The method according to any of claims 1 to 5, wherein a measured level above the reference level indicates that the risk is increased.

7. The method according to claim 6, wherein the increased risk relates to an increase of at least 1.5 times as compared to the risk of an average patient, preferably of an average patient of the same age, gender, and anemia causing disease.

8. The method according to any of claims 6 to 7, wherein the increased risk indicates that the patient should not be treated with a high dosage of an ESA wherein the high dosage of the ESA corresponds to a dosage sufficient to reach a hemoglobin concentration in blood of at least 14 pg/dl.

9. The method according to any of claims 1 to 8, wherein the ESA is chosen from the group consisting of (a) erythropoetin isolated from urine, (b) epoetin beta, (c) epoetin delta, (e) epoetin omega, (f) darbepoetin alpha, and (g) Cera.

10. The method according to any of claims 1 to 9, wherein the cardiovascular complication is coronary heart disease, acute coronary syndrome, myocardial infarction, left ventricular dysfunction, congestive heart failure, or thrombosis.

11. The method according to any of claims 1 to 10, wherein the level of the BNP-type peptide is measured using a specifically binding ligand, preferably an antibody or an aptamer.

12. Use of a diagnostic means for diagnosing the patient's risk of experiencing a cardiovascular complication as a consequence of future medication with ESA by in vitro measuring a patients level of a BNP-type peptide, particularly NT-pro BNP.

13. Use of a kit for diagnosing the risk of a patient of experiencing a cardiovascular complication as a consequence of future medication with ESA by in vitro measuring a patient's level of a BNP-type peptide, particularly NT-proBNP, the kit comprising means capable for measuring in vitro a patient's level of a BNP-type peptide, particularly NT-proBNP.

14. Use of a device for diagnosing the risk of a patient of experiencing a cardiovascular complication as a consequence of future medication with ESA by in vitro measuring a patients level of a BNP-type peptide, particularly NT-pro BNP, the device comprising
(a) means for measuring the amount of a BNP-type peptide thereof in a sample from the patient; and
(b) means for diagnosing said risk by comparing the measured level to a reference level.

15. A method for deciding on future medication of a patient with an ESA, particularly with a high dosage of an ESA, comprising the steps of
(a) measuring, in vitro, the level of a BNP-type peptide,
(b) diagnosing the risk of the patient of experiencing a cardiovascular complication as a consequence of the planned medication by comparing the measured level of the BNP-type peptide or a variant thereof to at least one reference level,
(c) optionally initiating an examination of the patient by a cardiologist,
(d) recommending to refrain from the ESA medication or recommending only medication with a low dosage of the ESA if the method indicates the presence of an increased risk of experiencing a cardiovascular complication as a consequence of the ESA medication.

## Patentansprüche

1. Verfahren zur Diagnose des Risikos eines Patienten, eine kardiovaskuläre Komplikation zu erleiden als Folge einer zukünftigen medikamentösen Behandlung mit einem Wirkstoff, der die Erythropoese fördert (ESA), umfassend die Schritte
a) Bestimmen des Spiegels eines Peptids des BNP-Typs in einer von dem Patienten stammenden Probe,
b) Diagnose des besagten Risikos durch Vergleich des bestimmten Spiegels des Peptids des BNP-Typs mit zumindest einem Referenzspiegel.

2. Verfahren nach Anspruch 1, wobei Schritt a) und/oder Schritt b) ausgeführt werden, bevor die medikamentöse Behandlung mit dem ESA beginnt und/oder wobei der Patient noch keine medikamentöse Behandlung mit einem ESA erfahren hat.

3. Verfahren nach einem der Ansprüche 1 oder 2, wobei das Peptid des BNP-Typs BNP oder NT-proBNP ist.

4. Verfahren nach Anspruch 3, wobei das Peptid des BNP-Typs BNP ist.

5. Verfahren nach einem der Ansprüche 1 bis 4, wobei der Referenzspiegel einem Plasmaspiegel von NT-proBNP von 300 bis 500 pg/ml entspricht.

6. Verfahren nach einem der Ansprüche 1 bis 5, wobei ein gemessener Spiegel oberhalb des Referenzspiegels ein erhöhtes Risiko anzeigt.

7. Verfahren nach Anspruch 6, wobei das erhöhte Risiko sich auf ein Risiko bezieht, das zumindest 1,5 mal erhöht ist verglichen mit dem Risiko eines durchschnittlichen Patienten, vorzugsweise eines durchschnittlichen Patienten des selben Alters, Geschlechts, und der selben Anämie verursachenden Krankheit.

8. Verfahren nach einem der Ansprüche 6 bis 7, wobei das erhöhte Risiko anzeigt, dass der Patient nicht mit einer hohen Dosierung eines ESA behandelt werden soll, wobei die hohe Dosierung des ESA einer Dosierung entspricht, die zum Erzielen einer Hämoglobin-Konzentration in Blut von wenigstens 14 pg/ml ausreichend ist.

9. Verfahren nach einem der Ansprüche 1 bis 8, wobei das ESA ausgewählt ist aus der Gruppe bestehend aus (a) aus Urin isoliertem Erythropoetin, (b) Epoetin beta, (c) Epoetin delta, (e) Epoetin omega, (f) Darbepoetin alpha, und (g) Cera.

10. Verfahren nach einem der Ansprüche 1 bis 9, wobei die kardiovaskuläre Komplikation ist: koronare Herzkrankheit, akutes Koronarsyndrom, Myokardinfarkt, linksventrikuläre Dysfunktion, kongestives Herzversagen, oder Thrombose.

11. Verfahren nach einem der Ansprüche 1 bis 10, wobei der Spiegel des Peptids des BNP-Typs unter Verwendung eines spezifisch bindenden Liganden bestimmt wird, volzugsweise eines Antokörpers oder eines Aptamers.

12. Verwendung eines diagnostischen Mittels zur Diagnose des Risikos eines Patienten, eine kardiovaskuläre Komplikation als Folge einer zukünftigen medikamentösen Behandlung mit einem ESA zu erleiden, durch *in vitro* Bestimmung des Spiegels eines Peptids des BNP-Typs, insbesondere NT-proBNP.

13. Verwendung eines Kits zur Diagnose des Risikos eines Patienten, eine kardiovaskuläre Komplikation als Folge einer zukünftigen medikamentösen Behandlung mit einem ESA zu erleiden, durch *in vitro* Bestimmung des Spiegels in einem Patienten eines Peptids des BNP-Typs, insbesondere NT-proBNP, wobei das Kit zur *in vitro* Bestimmung des Spiegel eines Peptids des BNP-Typs, insbesondere NT-proBNP, in einem Patienten geeignete Mittel enthält.

14. Verwendung einer Vorrichtung zur Diagnose des Risikos eines Patienten, eine kardiovaskuläre Komplikation als Folge einer zukünftigen medikamentösen Behandlung mit einem ESA zu erleiden, durch *in vitro* Bestimmung des Spiegels in einem Patienten eines Peptids des BNP-Typs, insbesondere NT-proBNP, wobei die Vorrichtung aufweist
(a) Mittel zur Messung der Menge eines Peptids des BNP-Typs in einer Probe des Patienten
(b) Mittel zur Diagnose des besagten Risikos durch Vergleich des gemessenen Spiegels mit einem Referenzspiegel.

15. Verfahren zur Entscheidung über die zukünftige medikamentöse Behandlung eines Patienten mit einem ESA, insbesondere einer hohen Dosierung eines ESA, enthaltend die Schritte
(a) *in vitro* Bestimmung des Spiegels eines Peptids des BNP-Typs,
(b) Diagnose des Risikos des Patienten, eine kardiovaskuläre Komplikation als Folge der geplanten medikamentösen Behandlung zu erleiden, durch Vergleich des gemessenen Spiegels des Peptids des BNP-Typs oder einer Variante davon mit mindestens einem Referenzwert,
(c) optionsweise Einleiten einer Untersuchung des Patienten durch einen Kardiologen,
(d) empfehlen, von der medikamentösen Behandlung mit ESA abzusehen oder empfehlen, nur eine medikamentöse Behandlung mit einer niedrigen Dosierung des ESA durchzuführen, wenn das Verfahren das Vorhandensein eines erhöhten Risikos anzeigt, eine kardiovaskuläre Komplikation als Folge der medikamentösen Behandlung mit ESA zu erleiden.

## Revendications

1. Procédé de diagnostic du risque pour un patient d'expérimenter une complication cardiovasculaire en conséquence d'un traitement médicamenteux futur à l'aide d'un agent de stimulation de l'érythropoïèse (ESA) comprenant les étapes de
a) mesure du taux de peptide de type BNP dans un prélèvement provenant du patient,
b) diagnostic dudit risque par comparaison du taux mesuré de peptide de type BNP ou d'un variant de celui-ci par rapport à au moins un taux de référence.

2. Procédé selon la revendication 1, l'étape a) et/ou l'étape b) étant conduite(s) avant le traitement médicamenteux avant que l'ESA ne commence et/ou dans lequel le patient n'a pas encore reçu de traitement médicamenteux avec un ESA.

3. Procédé selon la revendication 1 à 2, dans lequel le peptide de type BNP est BNP, NT-proBNP.

4. Procédé selon la revendication 3, le peptide de type BNP étant NT-proBNP.

5. Procédé selon l'une quelconque des revendications 1 à 4, le taux de référence correspondant à un taux plasmatique de NT-proBNP de 300 à 500 pg/ml.

6. Procédé selon l'une quelconque des revendications 1 à 5, dans lequel un taux mesuré situé au-dessus du taux de référence indique que le risque est accru.

7. Procédé selon la revendication 6, le risque accru étant lié à une augmentation d'au moins 1,5 fois tel que comparé au risque chez un patient moyen, de préférence chez un patient moyen du même âge, du même genre et de la même anémie provoquant la maladie.

8. Procédé selon l'une quelconque des revendications 6 à 7, le risque accru indiquant que le patient ne devrait pas être traité avec une dose élevée d'un ESA dans lequel la dose élevée de l'ESA correspond à une dose suffisante pour atteindre une concentration d'hémoglobine dans le sang d'au moins 14 pg/dl.

9. Procédé selon l'une quelconque des revendications 1 à 8, l'ESA étant choisi parmi le groupe constitué de (a) l'érythropoïétine isolée de l'urine, (b) l'époétine bêta, (c) l'époétine delta, (e) l'époétine oméga, (f) la darbépoétine alpha, et (g) C.E.R.A.

10. Procédé selon l'une quelconque des revendications 1 à 9, la complication cardiovasculaire étant l'insuffisance coronaire, le syndrome coronarien aigu, l'infarctus du myocarde, le dysfonctionnement du ventricule gauche, l'insuffisance cardiaque congestive ou la thrombose.

11. Procédé selon l'une quelconque des revendications 1 à 10, le taux de peptide de type BNP étant mesuré en utilisant un ligand se liant spécifiquement, de préférence un anticorps ou un aptamère.

12. Utilisation d'un moyen de diagnostic pour le diagnostic du risque pour un patient d'expérimenter une complication cardiovasculaire en conséquence d'un traitement médicamenteux futur avec de l'ESA par mesure *in vitro* du taux chez un patient d'un peptide de type BNP, particulièrement de NT-proBNP.

13. Utilisation d'une trousse pour le diagnostic du risque pour un patient d'expérimenter une complication cardiovasculaire en conséquence d'un traitement médicamenteux futur avec de l'ESA par mesure *in vitro* d'un taux chez un patient d'un peptide de type BNP, particulièrement NT-proBNP, la trousse comprenant des moyens capables de mesurer *in vitro* un taux chez un patient d'un peptide de type BNP, particulièrement NT-proBNP.

14. Utilisation d'un dispositif de diagnostic du risque pour un patient d'expérimenter une complication cardiovasculaire en conséquence d'un traitement médicamenteux futur avec de l'ESA par mesure *in vitro* d'un taux chez un patient d'un peptide de type BNP, particulièrement NT-proBNP, le dispositif comprenant
(a) des moyens de mesure de la quantité d'un peptide de type BNP dans un échantillon provenant d'un patient ; et
(b) des moyens de diagnostic dudit risque en comparant le taux mesuré à un taux de référence.

15. Procédé d'aide à la décision lors du choix d'un traitement médicamenteux futur par un ESA destiné à un patient, particulièrement avec une dose élevée d'un ESA, comprenant les étapes de
(a) mesure, *in vitro,* du taux d'un peptide de type BNP,
(b) diagnostic du risque pour un patient d'expérimenter une complication cardiovasculaire en conséquence d'un traitement médicamenteux planifié en comparant le taux mesuré du peptide de type BNP ou d'un de ses variants à au moins un taux de référence,
(c) initiation facultativement d'un examen du patient par un cardiologue,
(d) recommandation de s'abstenir du traitement médicamenteux par l'ESA ou recommandation uniquement d'un traitement médicamenteux avec une faible dose de l'ESA si le procédé indique la présence d'un risque accru de connaître une complication cardiovasculaire en conséquence du traitement médicamenteux par l'ESA.
